# EUROPÄISCHE PATENTSCHRIFT

(11) **EP 1 203 021 B1**
(45) Veröffentlichungstag und Bekanntmachung des Hinweises auf die Patenterteilung: **06.07.2005**
(21) Anmeldenummer: 00962210.1
(22) Anmeldetag: 04.08.2000
(51) Int. Cl.: C07K 14/705, C12N 15/12, C12Q 1/68

(54) **SEQUENZVARIANTEN DES MENSCHLICHEN BETA-1-ADRENOZEPTORGENS UND IHRE VERWENDUNG**
NEW SEQUENCE VARIANTS OF THE HUMAN BETA-1-ADRENORECEPTOR GENES AND USE THEREOF
NOUVEAUX VARIANTS DE SEQUENCES DU GENE HUMAIN DE L'ADRENORECEPTEUR BETA-1

(30) Priorität: 05.08.1999 DE 19938390
(43) Veröffentlichungstag der Anmeldung: 08.05.2002
(73) Patentinhaber: MAX-DELBRÜCK-CENTRUM FÜR MOLEKULARE MEDIZIN, 13125 Berlin (DE)
(72) Erfinder: WALLUKAT, Gerd, D-13129 Berlin (DE); PODLOWSKI, Svenja, D-15831 Mahlow (DE); WENZEL, Katrin, D-16321 Schönow (DE); MÜLLER, Johannes, D-10717 Berlin (DE)
(74) Vertreter: Baumbach, Friedrich, Dr.
(86) Internationale Anmeldenummer: PCT/DE2000/002648
(87) Internationale Veröffentlichungsnummer: WO 2001/011039

(56) Entgegenhaltungen:
- WO-A-00/22166
- WO-A-99/19512
- TESSON ET AL: "Characterization of a unique genetic variant in the beta-1-adrenoceptor gene and evaluation of its role in idiopathic dilated cardiomyopathy " J MOL CELL CARDIOL, Bd. 31, Mai 1999 (1999-05), Seiten 1025-1032, XP002163859
- MAQBOOL ET AL: "Common polymorphisms of beta-1-adrenoceptor: identification and rapid screening assay" THE LANCET, Bd. 353, März 1999 (1999-03), Seite 897 XP002163860 in der Anmeldung erwähnt
- MASON ET AL.: "A gain-of-function polymorphism in a G-protein coupling domain of the human beta-1-adrenergic receptor" JOURNAL OF BIOLOGICAL CHEMISTRY, Bd. 274, April 1999 (1999-04), Seiten 12670-12674, XP002163861
- PODLOWSKI ET AL.: J. MOL. MED, Bd. 78, 17. Februar 2000 (2000-02-17), Seiten 87-93, XP000983030

### SEQUENZPROTOKOLL

(1) ALLGEMEINE INFORMATION:
   (i) ANMELDER:
      (A) NAME: Max-Delbrueck-Centrum fuer Molekulare Medizin
      (B) STRASSE: Robert-Roessle-Str. 10
      (C) ORT: Berlin
      (E) LAND: Deutschland
      (F) POSTLEITZAHL: D-13125
   (ii) ANMELDETITEL: Neue Sequenzvarianten des menschlichen betal-Adrenozeptorgens und ihre Verwendung
   (iii) ANZAHL DER SEQUENZEN: 9
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
   (2) INFORMATION ZU SEQ ID NO: 1:
      (i) SEQUENZ CHARAKTERISTIKA
         (A) LÄNGE: 1637 Basenpaare
         (B) ART: Nukleinsäure
         (C) STRANGFORM: Doppel
         (D) TOPOLOGIE: linear
      (ii) ART DES MOLEKÜLS: DNS (genomisch)
      (xi) SEQUENZBESCHREIBUNG: SEQ ID NO: 1:
   ATGGGCGCGG GGGTGCTCGT CCTGGGCGCC TCCGAGCCCG GTAACCTGTC GTCGGCCGCA     60
   CCGCTCCCCG ACGGCGCGGC CACCGCGGCG CGGCTGCTGG TGCCCGCGTC GCCGCCCGCC    120
   TCGTTGCTGC CTCCCGCCAG CGAAGGCCCC GAGCCGCTGT CTCAGCAGTG GACATCGGGC    180
   ATGGGTCTGC TGATGGCGCT CATCGTGCTG CTCATCGTGG CGGGCAATGT GCTGGTGATC    240
   GTGGCCATCG CCAAGACGCC GCGGCTGCAG ACGCTCACCA ACCTCTTCAT CATGTCCCTG    300
   GCCAGCGCCG ACCTGGTCAT GGGGCTGCTG GTGGTGCCGT TCGGGGCCAC CATCGTGGTG    360
   TGGGGCCGCT GGGAGTACGG CTCCTTCTTC TGCGAGCTGT GGACCTCAGT GGACGTGCTG    420
   TGCGTGACGG CCAGCATCGA GACCCTGTGT GTCATTGCCC TGGACCGCTA CCTCGCCATC    480
   ACCTCGCCCT TCCGCTACCA GAGCCTGCTG ACGCGCGCGC GGGCGCGGGG CCTCGTGTGC    540
   ACCGTGTGGG CCATCTCGGC CCTGGTGTCC TTCCTGCCCA TCCTCATGCA CTGGTGGCGG     600
   GCGGAGAGCG ACGAGGCGCG CCGCTGCTAC AACGACCCCA AGTGCTGCGA CTTCGTCACC     660
   AACCGGGCCT ACGCCATCGC CTCGTCCGTA GTCTCCTTCT ACGTGCCCCT GTGCATCATG     720
   GCCTTCGTGT ACCTGCGGGT GTTCCGCGAG GCCCAGAAGC AGGTGAAGAA GATCGACAGC     780
   TGCGAGCGCC GTTTCCTCGG CGGCCCAGCG CGGCCGCCCT CGCCCTCGCC CTCGCCCGTC     840
   CCCGCGCCCG CGCCGCCGCC CGGACCCCCG CGCCCCGCCG CCGCCGCCGC CACCGCCCCG     900
   CTGGCCAACG GGCGTGCGGG TAAGCGGCGG CCCTCGCGCC TCGTGGCCCT ACGCGAGCAG     960
   AAGGCGCTCA AGACGCTGGG CATCATCATG GGCGTCTTCA CGCTCTGCTG GCTGCCCTTC    1020
   TTCCTGGCCA ACGTGGTGAA GGCCTTCCAC CGCGAGCTGG TGCCCGACCG CCTCTTCGTC    1080
   TTCTTCAACT GGCTGGGCTA CGCCAACTCG GCCTTCAACC CCATCATCTA CTGCCGCAGC    1140
   CCCGACTTCC GCAAGGCCTT CCAGCGACTG CTCTGCTGCG CGCGCAGGGC TGCCTGCCGG    1200
   CGCCGCGCGG CCCACGGAGA CCGGCCGCGC GCCTCGGGCT GTCTGGCCCG GGCCGGACCC    1260
   CCGCCATCGC CCGGGGCCGC CTCGGACGAC GACGACGACG ATGTCGTCGG GGCCACGCCG    1320
   CCCGCGCGCC TGCTGGAGCC CTGGGCCGGC TGCAACGGCG GGGCGGCGGC GGACAGCGAC    1380
   TCGAGCCTGG ACGAGCCGTG CCGCCCCGGC TTCGCCTCGG AATCCAAGGT GTAGGGCCCG    1440
   GCGCGGGGCG CGGACTCCGG GCACGGCTTC CCAGGGGAAC GAGGAGATCT GTGTTTACTT    1500
   AAGACCGATA GCAGGTGAAC TCGAAGCCCA CAATCCTCGT CTGAATCATC CGAGGCAAAG    1560
   AGAAAAGCCA CGGACCGTTG CACAAAAAGG AAAGTTTGGG AAGGGATGGG AGAGTGGCTT    1620
   GCTGATGTTC CTTGTTG                                                  1637
(2) INFORMATION ZU SEQ ID NO: 2:
   (i) SEQUENZ CHARAKTERISTIKA:
      (A) LÄNGE: 1637 Basenpaare
      (B) ART: Nukleinsäure
      (C) STRANGFORM: Doppel
      (D) TOPOLOGIE: linear
   (ii) ART DES MOLEKÜLS: DNS (genomisch)
   (xi) SEQUENZBESCHREIBUNG: SEQ ID NO: 2:
   ATGGGCGCGG GGGTGCTCGT CCTGGGCGCC TCCGAGCCCG GTAACCTGTC GTCGGCCGCA      60
   CCGCTCCCCG ACGGCGCGGC CACCGCGGCG CGGCTGCTGG TGCCCGCGTC GCCGCCCGCC     120
   TCGTTGCTGC CTCCCGCCAG CGAAGGCCCC GAGCCGCTGT CTCAGCAGTG GACAGCGGGC     180
   ATGGGTCTGC TGATGGCGCT CATCGTGCTG CTCATCGTGG CGGGCAATGT GCTGGTGATC     240
   GTGGCCATCG CCAAGACGCC GCGGCTGCAG ACGCTCACCA ACCTCTTCAT CATGTCCCTG     300
   GCCAGCGCCG ACCTGGTCAT GGGGCTGCTG GTGGTGCCGT TCGGGGCCAC CATCGTGGTG     360
   TGGGGCCGCT GGGAGTACGG CTCCTTCTTC TGCGAGCTGT GGACCTCAGT GGACGTGCTG     420
   TGCGTGACGG CCAGCATCGA GACCCTGTGT GTCATTGCCC TGGACCGCTA CCTCGCCATC     480
   ACCTCGCCCT TCCGCTACCA GAGCCTGCTG ACGCGCGCGC GGGCGCGGGG CCTCGTGTGC     540
   ACCGTGTGGG CCATCTCGGC CCTGGTGTCC TTCCTGCCCA TCCTCATGCA CTGGTGGCGG     600
   GCGGAGAGCG ACGAGGCGCG CCGCTGCTAC AACGACCCCA AGTGCTGCGA CTTCGTCACC     660
   AACCGGGCCT ACGCCATCGC CTCGTCCGTA GTCTCCTTCT ACGTGCCCCT GTGCATCATG     720
   GCCTTCGTGT ACCTGCGGGT GTTCCGCGAG GCCCAGAAGC AGGTGAAGAA GATCGACAGC     780
   TGCGAGCGCC GTTTCCTCGG CGGCCCAGCG CGGCCGCCCT CGCCCTCGCC CTCGCCCGTC     840
   CCCGCGCCCG CGCCGCCGCC CGGACCCCCG CGCCCCGCCG CCGCCGCCGC CACCGCCCCG     900
   CTGGCCAACG GGCGTGCGGG TAAGCGGCGG CCCTCGCGCC TCGTGGCCCT ACGCGAGCAG     960
   AAGGCGCTCA AGACGCTGGG CATCATCATG GGCGTCTTCA CGCTCTGCTG GCTGCCCTTC    1020
   TTCCTGGCCA ACGTGGTGAA GGCCTTCCAC CGCGAGCTGG TGCCCGACCG CCTCTTCGTC    1080
   TTCTTCAACT GGCTGGGCTA CGCCAACTCG GCCTTCAACC CCATCATCTA CTGCCGCAGC    1140
   CCCGACTTCC GCAAGGCCTT CCAGGGACTG CTCTGCTGCG CGCGCAGGGC TGCCCGCCGG    1200
   CGCCACGCGA CCCACGGAGA CCGGCCGCGC GCCTCGGGCT GTCTGGCCCG GCCCGGACCC    1260
   CCGCCATCGC CCGGGGCCGC CTCGGACGAC GACGACGACG ATGTCGTCGG GGCCACGCCG    1320
   CCCGCGCGCC TGCTGGAGCC CTGGGCCGGC TGCAACGGCG GGGCGGCGGC GGACAGCGAC    1380
   TCGAGCCTGG ACGAGCCGTG CCGCCCCGGC TTCGCCTCGG AATCCAAGGT GTAGGGCCCG    1440
   GCGCGGGGCG CGGACTCCGG GCACGGCTTC CCAGGGGAAC GAGGAGATCT GTGTTTACTT    1500
   AAGACCGATA GCAGGTGAAC TCGAAGCCCA CAATCCTCGT CTGAATCATC CGAGGCAAAG    1560
   AGAAAAGCCA CGGACCGTTG CACAAAAAGG AAAGTTTGGG AAGGGATGGG AGAGTGGCTT    1620
   GCTGATGTTC CTTGTTG                                                   1637
(2) INFORMATION ZU SEQ ID NO: 3:
   (1) SEQUENZ CHARAKTERISTIKA:
      (A) LÄNGE: 1637 Basenpaare
      (B) ART: Nukleinsäure
      (C) STRANGFORM: Doppel
      (D) TOPOLOGIE. linear
   (ii) ART DES MOLEKÜLS: DNS (genomisch)
   (xi) SEQUENZBESCHREIBUNG: SEQ ID NO: 3:
   ATGGGCGCGG GGGTGCTCGT CCTGGGCGCC TCCGAGCCCG GTAACCTGTC GTCGGCCGCA     60
   CCGCTCCCCG ACGGCGCGGC CACCGCGGCG CGGCTGCTGG TGCCCGCGTC GCCGCCCGCC    120
   TCGTTGCTGC CTCCCGCCAG CGAAAGCCCC GAGCCGCTGT CTCAGCAGTG GACATCGGGC    180
   ATGGGTCTGC TGATGGCGCT CATCGTGCTG CTCATCGTGG CGGGCAATGT GCTGGTGATC    240
   GTGGCCATCG CCAAGACGCC GCGGCTGCAG ACGCTCACCA ACCTCTTCAT CATGTCCCTG    300
   GCCAGCGCCG ACCTGGTCAT GGGGCTGCTG GTGGTGCCGT TCGGGGCCAC CATCGTGGTG    360
   TGGGGCCGCT GGGAGTACGG CTCCTTCTTC TGCGAGCTGT GGACCTCAGT GGACGTGCTG    420
   TGCGTGACGG CCAGCATCGA GACCCTGTGT GTCATTGCCC TGGACCGCTA CCTCGCCATC    480
   ACCTCGCCCT TCCGCTACCA GAGCCTGCTG ACGCGCGCGC GGGCGCGGGG CCTCGTGTGC    540
   ACCGTGTGGG CCATCTCGGC CCTGGTGTCC TTCCTGCCCA TCCTCATGCA CTGGTGGCGG    600
   GCGGAGAGCG ACGAGGCGCG CCGCTGCTAC AACGACCCCA AGTGCTGCGA CTTCGTCACC    660
   AACCGGGCCT ACGCCATCGC CTCGTCCGTA GTCTCCTTCT ACGTGCCCCT GTGCATCATG    720
   GCCTTCGTGT ACCTGCGGGT GTTCCGCGAG GCCCAGAAGC AGGTGAAGAA GATCGACAGC    780
   TGCGAGCGCC GTTTCCTCGG CGGCCCAGCG CGGCCGCCCT CGCCCTCGCC CTCGCCCGTC    840
   CCCGCGCCCG CGCCGCCGCC CGGACCCCCG CGCCCCGCCG CCGCCGCCGC CACCGCCCCG    900
   CTGGCCAACG GGCGTGCGGG TAAGCGGCGG CCCTCGCGCC TCGTGGCCCT ACGCGAGCAG    960
   AAGGCGCTCA AGACGCTGGG CATCATCATG GGCGTCTTCA CGCTCTGCTG GCTGCCCTTC   1020
   TTCCTGGCCA ACGTGGTGAA GGCCTTCCAC CGCGAGCTGG TGCCCGACCG CCTCTTCGTC   1080
   TTCTTCAACT GGCTGGGCTA CGCCAACTCG GCCTTCAACC CCATCATCTA CTGCCGCAGC   1140
   CCCGACTTCC GCAAGGCCTT CCAGGGACTG CTCTGCTGCG CGCGCAGGGC TGCCCGCCGG   1200
   CGCCACGCGA CCCACGGAGA CCGGCCGCGC GCCTCGGGCT GTCTGGCCCG GCCCGGACCC   1260
   CCGCCATCGC CCGGGGCCGC CTCGGACGAC GACGACGACG ATGTCGTCGG GGCCACGCCG   1320
   CCCGCGCGCC TGCTGGAGCC CTGGGCCGGC TGCAACGGCG GGGCGGCGGC GGACAGCGAC   1380
   TCGAGCCTGG ACGAGCCGTG CCGCCCCGGC TTCGCCTCGG AATCCAAGGT GTAGGGCCCG   1440
   GCGCGGGGCG CGGACTCCGG GCACGGCTTC CCAGGGGAAC GAGGAGATCT GTGTTTACTT   1500
   AAGACCGATA GCAGGTGAAC TCGAAGCCCA CAATCCTCGT CTGAATCATC CGAGGCAAAG   1560
   AGAAAAGCCA CGGACCGTTG CACAAAAAGG AAAGTTTGGG AAGGGATGGG AGAGTGGCTT   1620
   GCTGATGTTC CTTGTTG                                                  1637
(2) INFORMATION ZU SEQ ID NO: 4:
   (i) SEQUENZ CHARAKTERISTIKA:
      (A) LÄNGE: 1637 Basenpaare
      (B) ART: Nukleinsäure
      (C) STRANGFORM: Doppel
      (D) TOPOLOGIE: linear
   (ii) ART DES MOLEKÜLS: DNS (genomisch)
   (xi) SEQUENZBESCHREIBUNG: SEQ ID NO: 4:
   ATGGGCGCGG GGGTGCTCGT CCTGGGCGCC TCCGAGCCCG GTAACCTGTC GTCGGCCGCA      60
   CCGCTCCCCG ACGGCGCGGC CACCGCGGCG CGGCTGCTGG TGCCCGCGTC GCCGCCCGCC     120
   TCGTTGCTGC CTCCCGCCAG CGAAAGCCCC GAGCCGCTGT CTCAGCAGTG GACAGCGGGC     180
   ATGGGTCTGC TGATGGCGCT CATCGTGCTG CTCATCGTGG CGGGCAATGT GCTGGTGATC     240
   GTGGCCATCG CCAAGACGCC GCGGCTGCAG ACGCTCACCA ACCTCTTCAT CATGTCCCTG     300
   GCCAGCGCCG ACCTGGTCAT GGGGCTGCTG GTGGTGCCGT TCGGGGCCAC CATCGTGGTG     360
   TGGGGCCGCT GGGAGTACGG CTCCTTCTTC TGCGAGCTGT GGACCTCAGT GGACGTGCTG     420
   TGCGTGACGG CCAGCATCGA GACCCTGTGT GTCATTGCCC TGGACCGCTA CCTCGCCATC     480
   ACCTCGCCCT TCCGCTACCA GAGCCTGCTG ACGCGCGCGC GGGCGCGGGG CCTCGTGTGC     540
   ACCGTGTGGG CCATCTCGGC CCTGGTGTCC TTCCTGCCCA TCCTCATGCA CTGGTGGCGG     600
   GCGGAGAGCG ACGAGGCGCG CCGCTGCTAC AACGACCCCA AGTGCTGCGA CTTCGTCACC     660
   AACCGGGCCT ACGCCATCGC CTCGTCCGTA GTCTCCTTCT ACGTGCCCCT GTGCATCATG     720
   GCCTTCGTGT ACCTGCGGGT GTTCCGCGAG GCCCAGAAGC AGGTGAAGAA GATCGACAGC     780
   TGCGAGCGCC GTTTCCTCGG CGGCCCAGCG CGGCCGCCCT CGCCCTCGCC CTCGCCCGTC     840
   CCCGCGCCCG CGCCGCCGCC CGGACCCCCG CGCCCCGCCG CCGCCGCCGC CACCGCCCCG     900
   CTGGCCAACG GGCGTGCGGG TAAGCGGCGG CCCTCGCGCC TCGTGGCCCT ACGCGAGCAG     960
   AAGGCGCTCA AGACGCTGGG CATCATCATG GGCGTCTTCA CGCTCTGCTG GCTGCCCTTC    1020
   TTCCTGGCCA ACGTGGTGAA GGCCTTCCAC CGCGAGCTGG TGCCCGACCG CCTCTTCGTC    1080
   TTCTTCAACT GGCTGGGCTA CGCCAACTCG GCCTTCAACC CCATCATCTA CTGCCGCAGC    1140
   CCCGACTTCC GCAAGGCCTT CCAGCGACTG CTCTGCTGCG CGCGCAGGGC TGCCCGCCGG    1200
   CGCCACGCGA CCCACGGAGA CCGGCCGCGC GCCTCGGGCT GTCTGGCCCG GCCCGGACCC    1260
   CCGCCATCGC CCGGGGCCGC CTCGGACGAC GACGACGACG ATGTCGTCGG GGCCACGCCG    1320
   CCCGCGCGCC TGCTGGAGCC CTGGGCCGGC TGCAACGGCG GGGCGGCGGC GGACAGCGAC    1380
   TCGAGCCTGG ACGAGCCGTG CCGCCCCGGC TTCGCCTCGG AATCCAAGGT GTAGGGCCCG    1440
   GCGCGGGGCG CGGACTCCGG GCACGGCTTC CCAGGGGAAC GAGGAGATCT GTGTTTACTT    1500
   AAGACCGATA GCAGGTGAAC TCGAAGCCCA CAATCCTCGT CTGAATCATC CGAGGCAAAG    1560
   AGAAAAGCCA CGGACCGTTG CACAAAAAGG AAAGTTTGGG AAGGGATGGG AGAGTGGCTT    1620
   GCTGATGTTC CTTGTTG                                                   1637
(2) INFORMATION ZU SEQ ID NO: 5:
   (i) SEQUENZ CHARAKTERISTIKA:
      (A) LÄNGE: 1637 Basenpaare
      (B) ART: Nukleinsäure
      (C) STRANGFORM Doppel
      (D) TOPOLOGIE: linear
   (ii) ART DES MOLEKÜLS: DNS (genomisch)
   (xi) SEQUENZBESCHREIBUNG: SEQ ID NO: 5:
   ATGGGCGCGG GGGTGCTCGT CCTGGGCGCC TCCGAGCCCG GTAACCTGTC GTCGGCCGCA      60
   CCGCTCCCCG ACGGCGCGGC CACCGCGGCG CGGCTGCTGG TGCCCGCGTC GCCGCCCGCC     120
   TCGTTGCTGC CTCCCGCCAG CGAAGGCCCC GAGCCGCTGT CTCAGCAGTG GACATCGGGC     180
   ATGGGTCTGC TGATGGCGCT CATCGTGCTG CTCATCGTGG CGGGCAATGT GCTGGTGATC     240
   GTGGCCATCG CCAAGACGCC GCGGCTGCAG ACGCTCACCA ACCTCTTCAT CATGTCCCTG     300
   GCCAGCGCCG ACCTGGTCAT GGGGCTGCTG GTGGTGCCGT TCGGGGCCAC CATCGTGGTG     360
   TGGGGCCGCT GGGAGTACGG CTCCTTCTTC TGCGAGCTGT GGACCTCAGT GGACGTGCTG     420
   TGCGTGACGG CCAGCATCGA GACCCTGTGT GTCATTGCCC TGGACCGCTA CCTCGCCATC     480
   ACCTCGCCCT TCCGCTACCA GAGCCTGCTG ACGCGCGCGC GGGCGCGGGG CCTCGTGTGC     540
   ACCGTGTGGG CCATCTCGGC CCTGGTGTCC TTCCTGCCCA TCCTCATGCA CTGGTGGCGG     600
   GCGGAGAGCG ACGAGGCGCG CCGCTGCTAC AACGACCCCA AGTGCTGCGA CTTCGTCACC     660
   AACCGGGCCT ACGCCATCGC CTCGTCCGTA GTCTCCTTCT ACGTGCCCCT GTGCATCATG     720
   GCCTTCGTGT ACCTGCGGGT GTTCCGCGAG GCCCAGAAGC AGGTGAAGAA GATCGACAGC     780
   TGCGAGCGCC GTTTCCTCGG CGGCCCAGCG CGGCCGCCCT CGCCCTCGCC CTCGCCCGTC     840
   CCCGCGCCCG CGCCGCCGCC CGGACCCCCG CGCCCCGCCG CCGCCGCCGC CACCGCCCCG     900
   CTGGCCAACG GGCGTGCGGG TAAGCGGCGG CCCTCGCGCC TCGTGGCCCT ACGCGAGCAG     960
   AAGGCGCTCA AGACGCTGGG CATCATCATG GGCGTCTTCA CGCTCTGCTG GCTGCCCTTC    1020
   TTCCTGGCCA ACGTGGTGAA GGCCTTCCAC CGCGAGCTGG TGCCCGACCG CCTCTTCGTC    1080
   TTCTTCAACT GGCTGGGCTA CGCCAACTCG GCCTTCAACC CCATCATCTA CTGCCGCAGC    1140
   CCCGACTTCC GCAAGGCCTT CCAGGGACTG CTCTGCTGCG CGCGCAGGGC TGCCCGCCGG    1200
   CGCCACGCGA CCCACGGAGA CCGGCCGCGC GCCTCGGGCT GTCTGGCCCG GCCCGGACCC    1260
   CCGCCATCGC CCGGGGCCGC CTCGGACGAC GACGACGACG ATGTCGTCGG GGCCACGCCG    1320
   CCCGCGCGCC TGCTGGAGCC CTGGGCCGGC TGCAACGGCG GGGCGGCGGC GGACAGCGAC    1380
   TCGAGCCTGG ACGAGCCGTG CCGCCCCGGC TTCGCCTCGG AATCCAAGGT GTAGGGCCCG    1440
   GCGCGGGGCG CGGACTCCGG GCACGGCTTC CCAGGGGAAC GAGGAGATCT GTGTTTACTT    1500
   AAGACCGATA GCAGGTGAAC TCGAAGCCCA CAATCCTCGT CTGAATCATC CGAGGCAAAG    1560
   AGAAAAGCCA CGGACCGTTG CACAAAAAGG AAAGTTTGGG AAGGGATGGG AGAGTGGCTT    1620
   GCTGATGTTC CTTGTTG                                                   1637
(2) INFORMATION ZU SEQ ID NO: 6:
   (i) SEQUENZ CHARAKTERISTIKA:
      (A) LÄNGE: 1637 Basenpaare
      (B) ART: Nukleinsäure
      (C) STRANGFORM: Doppel
      (D) TOPOLOGIE: linear
   (ii) ART DES MOLEKÜLS: DNS (genomisch)
   (xi) SEQUENZBESCHREIBUNG: SEQ ID NO: 6:
   ATGGGCGCGG GGGTGCTCGT CCTGGGCGCC TCCGAGCCCG GTAACCTGTC GTCGGCCGCA     60
   CCGCTCCCCG ACGGCGCGGC CACCGCGGCG CGGCTGCTGG TGCCCGCGTC GCCGCCCGCC    120
   TCGTTGCTGC CTCCCGCCAG CGAAGGCCCC GAGCCGCTGT CTCAGCAGTG GACAGCGGGC    180
   ATGGGTCTGC TGATGGCGCT CATCGTGCTG CTCATCGTGG CGGGCAATGT GCTGGTGATC    240
   GTGGCCATCG CCAAGACGCC GCGGCTGCAG ACGCTCACCA ACCTCTTCAT CATGTCCCTG    300
   GCCAGCGCCG ACCTGGTCAT GGGGCTGCTG GTGGTGCCGT TCGGGGCCAC CATCGTGGTG    360
   TGGGGCCGCT GGGAGTACGG CTCCTTCTTC TGCGAGCTGT GGACCTCAGT GGACGTGCTG    420
   TGCGTGACGG CCAGCATCGA GACCCTGTGT GTCATTGCCC TGGACCGCTA CCTCGCCATC     480
   ACCTCGCCCT TCCGCTACCA GAGCCTGCTG ACGCGCGCGC GGGCGCGGGG CCTCGTGTGC     540
   ACCGTGTGGG CCATCTCGGC CCTGGTGTCC TTCCTGCCCA TCCTCATGCA CTGGTGGCGG     600
   GCGGAGAGCG ACGAGGCGCG CCGCTGCTAC AACGACCCCA AGTGCTGCGA CTTCGTCACC     660
   AACCGGGCCT ACGCCATCGC CTCGTCCGTA GTCTCCTTCT ACGTGCCCCT GTGCATCATG     720
   GCCTTCGTGT ACCTGCGGGT GTTCCGCGAG GCCCAGAAGC AGGTGAAGAA GATCGACAGC     780
   TGCGAGCGCC GTTTCCTCGG CGGCCCAGCG CGGCCGCCCT CGCCCTCGCC CTCGCCCGTC     840
   CCCGCGCCCG CGCCGCCGCC CGGACCCCCG CGCCCCGCCG CCGCCGCCGC CACCGCCCCG     900
   CTGGCCAACG GGCGTGCGGG TAAGCGGCGG CCCTCGCGCC TCGTGGCCCT ACGCGAGCAG     960
   AAGGCGCTCA AGACGCTGGG CATCATCATG GGCGTCTTCA CGCTCTGCTG GCTGCCCTTC    1020
   TTCCTGGCCA ACGTGGTGAA GGCCTTCCAC CGCGAGCTGG TGCCCGACCG CCTCTTCGTC    1080
   TTCTTCAACT GGCTGGGCTA CGCCAACTCG GCCTTCAACC CCATCATCTA CTGCCGCAGC    1140
   CCCGACTTCC GCAAGGCCTT CCAGCGACTG CTCTGCTGCG CGCGCAGGGC TGCCCGCCGG    1200
   CGCCACGCGA CCCACGGAGA CCGGCCGCGC GCCTCGGGCT GTCTGGCCCG GCCCGGACCC    1260
   CCGCCATCGC CCGGGGCCGC CTCGGACGAC GACGACGACG ATGTCGTCGG GGCCACGCCG    1320
   CCCGCGCGCC TGCTGGAGCC CTGGGCCGGC TGCAACGGCG GGGCGGCGGC GGACAGCGAC    1380
   TCGAGCCTGG ACGAGCCGTG CCGCCCCGGC TTCGCCTCGG AATCCAAGGT GTAGGGCCCG    1440
   GCGCGGGGCG CGGACTCCGG GCACGGCTTC CCAGGGGAAC GAGGAGATCT GTGTTTACTT    1500
   AAGACCGATA GCAGGTGAAC TCGAAGCCCA CAATCCTCGT CTGAATCATC CGAGGCAAAG    1560
   AGAAAAGCCA CGGACCGTTG CACAAAAAGG AAAGTTTGGG AAGGGATGGG AGAGTGGCTT    1620
   GCTGATGTTC CTTGTTG                                                   1637
(2) INFORMATION ZU SEQ ID NO: 7:
   (i) SEQUENZ CHARAKTERISTIKA
      (A) LÄNGE: 1637 Basenpaare
      (B) ART: Nukleinsäure
      (C) STRANGFORM: Doppel
      (D) TOPOLOGIE: linear
   (ii) ART DES MOLEKÜLS: DNS (genomisch)
   (xi) SEQUENZBESCHREIBUNG: SEQ ID NO: 7:
   ATGGGCGCGG GGGTGCTCGT CCTGGGCGCC TCCGAGCCCG GTAACCTGTC GTCGGCCGCA     60
   CCGCTCCCCG ACGGCGCGGC CACCGCGGCG CGGCTGCTGG TGCCCGCGTC GCCGCCCGCC    120
   TCGTTGCTGC CTCCCGCCAG CGAAAGCCCC GAGCCGCTGT CTCAGCAGTG GACATCGGGC    180
   ATGGGTCTGC TGATGGCGCT CATCGTGCTG CTCATCGTGG CGGGCAATGT GCTGGTGATC    240
   GTGGCCATCG CCAAGACGCC GCGGCTGCAG ACGCTCACCA ACCTCTTCAT CATGTCCCTG    300
   GCCAGCGCCG ACCTGGTCAT GGGGCTGCTG GTGGTGCCGT TCGGGGCCAC CATCGTGGTG    360
   TGGGGCCGCT GGGAGTACGG CTCCTTCTTC TGCGAGCTGT GGACCTCAGT GGACGTGCTG    420
   TGCGTGACGG CCAGCATCGA GACCCTGTGT GTCATTGCCC TGGACCGCTA CCTCGCCATC    480
   ACCTCGCCCT TCCGCTACCA GAGCCTGCTG ACGCGCGCGC GGGCGCGGGG CCTCGTGTGC    540
   ACCGTGTGGG CCATCTCGGC CCTGGTGTCC TTCCTGCCCA TCCTCATGCA CTGGTGGCGG    600
   GCGGAGAGCG ACGAGGCGCG CCGCTGCTAC AACGACCCCA AGTGCTGCGA CTTCGTCACC    660
   AACCGGGCCT ACGCCATCGC CTCGTCCGTA GTCTCCTTCT ACGTGCCCCT GTGCATCATG    720
   GCCTTCGTGT ACCTGCGGGT GTTCCGCGAG GCCCAGAAGC AGGTGAAGAA GATCGACAGC    780
   TGCGAGCGCC GTTTCCTCGG CGGCCCAGCG CGGCCGCCCT CGCCCTCGCC CTCGCCCGTC    840
   CCCGCGCCCG CGCCGCCGCC CGGACCCCCG CGCCCCGCCG CCGCCGCCGC CACCGCCCCG    900
   CTGGCCAACG GGCGTGCGGG TAAGCGGCGG CCCTCGCGCC TCGTGGCCCT ACGCGAGCAG    960
   AAGGCGCTCA AGACGCTGGG CATCATCATG GGCGTCTTCA CGCTCTGCTG GCTGCCCTTC   1020
   TTCCTGGCCA ACGTGGTGAA GGCCTTCCAC CGCGAGCTGG TGCCCGACCG CCTCTTCGTC   1080
   TTCTTCAACT GGCTGGGCTA CGCCAACTCG GCCTTCAACC CCATCATCTA CTGCCGCAGC   1140
   CCCGACTTCC GCAAGGCCTT CCAGCGACTG CTCTGCTGCG CGCGCAGGGC TGCCCGCCGG   1200
   CGCCACGCGA CCCACGGAGA CCGGCCGCGC GCCTCGGGCT GTCTGGCCCG GCCCGGACCC   1260
   CCGCCATCGC CCGGGGCCGC CTCGGACGAC GACGACGACG ATGTCGTCGG GGCCACGCCG   1320
   CCCGCGCGCC TGCTGGAGCC CTGGGCCGGC TGCAACGGCG GGGCGGCGGC GGACAGCGAC   1380
   TCGAGCCTGG ACGAGCCGTG CCGCCCCGGC TTCGCCTCGG AATCCAAGGT GTAGGGCCCG   1440
   GCGCGGGGCG CGGACTCCGG GCACGGCTTC CCAGGGGAAC GAGGAGATCT GTGTTTACTT   1500
   AAGACCGATA GCAGGTGAAC TCGAAGCCCA CAATCCTCGT CTGAATCATC CGAGGCAAAG   1560
   AGAAAAGCCA CGGACCGTTG CACAAAAAGG AAAGTTTGGG AAGGGATGGG AGAGTGGCTT   1620
   GCTGATGTTC CTTGTTG                                                  1637
(2) INFORMATION ZU SEQ ID NO: 8:
   (1) SEQUENZ CHARAKTERISTIKA:
      (A) LÄNGE: 1637 Basenpaare
      (B) ART: Nukleinsäure
      (C) STRANGFORM: Doppel
      (D) TOPOLOGIE: linear
   (ii) ART DES MOLEKÜLS: DNS (genomisch)
   (xi) SEQUENZBESCHREIBUNG: SEQ ID NO: 8:
   ATGGGCGCGG GGGTGCTCGT CCTGGGCGCC TCCGAGCCCG GTAACCTGTC GTCGGCCGCA     60
   CCGCTCCCCG ACGGCGCGGC CACCGCGGCG CGGCTGCTGG TGCCCGCGTC GCCGCCCGCC    120
   TCGTTGCTGC CTCCCGCCAG CGAAGGCCCC GAGCCGCTGT CTCAGCAGTG GACATCGGGC    180
   ATGGGTCTGC TGATGGCGCT CATCGTGCTG CTCATCGTGG CGGGCAATGT GCTGGTGATC    240
   GTGGCCATCG CCAAGACGCC GCGGCTGCAG ACGCTCACCA ACCTCTTCAT CATGTCCCTG    300
   GCCAGCGCCG ACCTGGTCAT GGGGCTGCTG GTGGTGCCGT TCGGGGCCAC CATCGTGGTG    360
   TGGGGCCGCT GGGAGTACGG CTCCTTCTTC TGCGAGCTGT GGACCTCAGT GGACGTGCTG    420
   TGCGTGACGG CCAGCATCGA GACCCTGTGT GTCATTGCCC TGGACCGCTA CCTCGCCATC    480
   ACCTCGCCCT TCCGCTACCA GAGCCTGCTG ACGCGCGCGC GGGCGCGGGG CCTCGTGTGC    540
   ACCGTGTGGG CCATCTCGGC CCTGGTGTCC TTCCTGCCCA TCCTCATGCA CTGGTGGCGG    600
   GCGGAGAGCG ACGAGGCGCG CCGCTGCTAC AACGACCCCA AGTGCTGCGA CTTCGTCACC    660
   AACCGGGCCT ACGCCATCGC CTCGTCCGTA GTCTCCTTCT ACGTGCCCCT GTGCATCATG    720
   GCCTTCGTGT ACCTGCGGGT GTTCCGCGAG GCCCAGAAGC AGGTGAAGAA GATCGACAGC    780
   TGCGAGCGCC GTTTCCTCGG CGGCCCAGCG CGGCCGCCCT CGCCCTCGCC CTCGCCCGTC    840
   CCCGCGCCCG CGCCGCCGCC CGGACCCCCG CGCCCCGCCG CCGCCGCCGC CACCGCCCCG    900
   CTGGCCAACG GGCGTGCGGG TAAGCGGCGG CCCTCGCGCC TCGTGGCCCT ACGCGAGCAG    960
   AAGGCGCTCA AGACGCTGGG CATCATCATG GGCGTCTTCA CGCTCTGCTG GCTGCCCTTC   1020
   TTCCTGGCCA ACGTGGTGAA GGCCTTCCAC CGCGAGCTGG TGCCCGACCG CCTCTTCGTC   1080
   TTCTTCAACT GGCTGGGCTA CGCCAACTCG GCCTTCAACC CCATCATCTA CTGCCGCAGC   1140
   CCCGACTTCC GCAAGGCCTT CCAGCGACTG CTCTGCTGCG CGCGCAGGGC TGCCCGCCGG   1200
   CGCCACGCGA CCCACGGAGA CCGGCCGCGC GCCTCGGGCT GTCTGGCCCG GCCCGGACCC   1260
   CCGCCATCGC CCGGGGCCGC CTCGGACGAC GACGACGACG ATGTCGTCGG GGCCACGCCG   1320
   CCCGCGCGCC TGCTGGAGCC CTGGGCCGGC TGCAACGGCG GGGCGGCGGC GGACAGCGAC   1380
   TCGAGCCTGG ACGAGCCGTG CCGCCCCGGC TTCGCCTCGG AATCCAAGGT GTAGGGCCCG    1440
   GCGCGGGGCG CGGACTCCGG GCACGGCTTC CCAGGGGAAC GAGGAGATCT GTGTTTACTT    1500
   AAGACCGATA GCAGGTGAAC TCGAAGCCCA CAATCCTCGT CTGAATCATC CGAGGCAAAG    1560
   AGAAAAGCCA CGGACCGTTG CACAAAAAGG AAAGTTTGGG AAGGGATGGG AGAGTGGCTT    1620
   GCTGATGTTC CTTGTTG                                                   1637
(2) INFORMATION ZU SEQ ID NO: 9:
   (1) SEQUENZ CHARAKTERISTIKA:
      (A) LÄNGE: 477 Aminosäuren
      (B) ART: Aminosäure
      (C) STRANGFORM: Einzel
      (D) TOPOLOGIE: linear
   (ii) ART DES MOLEKÜLS: Protein
   (xi) SEQUENZBESCHREIBUNG: SEQ ID NO: 9:
   Met Gly Ala Gly Val Leu Val Leu Gly Ala Ser Glu Pro Gly Asn Leu
   1               5                   10                  15
   Ser Ser Ala Ala Pro Leu Pro Asp Gly Ala Ala Thr Ala Ala Arg Leu
               20                  25                  30
   Leu Val Pro Ala Ser Pro Pro Ala Ser Leu Leu Pro Pro Ala Ser Glu
           35                  40                  45
   Gly Pro Glu Pro Leu Ser Gln Gln Trp Thr Ser Gly Met Gly Leu Leu
       50                  55                  60
   Met Ala Leu Ile Val Leu Leu Ile Val Ala Gly Asn Val Leu Val Ile
   65                  70                  75                  80
   Val Ala Ile Ala Lys Thr Pro Arg Leu Gln Thr Leu Thr Asn Leu Phe
                   85                  90                  95
   Ile Met Ser Leu Ala Ser Ala Asp Leu Val Met Gly Leu Leu Val Val
               100                 105                 110
   Pro Phe Gly Ala Thr Ile Val Val Trp Gly Arg Trp Glu Tyr Gly Ser
           115                 120                 125
   Phe Phe Cys Glu Leu Trp Thr Ser Val Asp Val Leu Cys Val Thr Ala
       130                 135                 140
   Ser Ile Glu Thr Leu Cys Val Ile Ala Leu Asp Arg Tyr Leu Ala Ile
   145                 150                 155                 160
   Thr Ser Pro Phe Arg Tyr Gln Ser Leu Leu Thr Arg Ala Arg Ala Arg
                   165                 170                 175
   Gly Leu Val Cys Thr Val Trp Ala Ile Ser Ala Leu Val Ser Phe Leu
               180                 185                 190
   Pro Ile Leu Met His Trp Trp Arg Ala Glu Ser Asp Glu Ala Arg Arg
           195                 200                 205
   Cys Tyr Asn Asp Pro Lys Cys Cys Asp Phe Val Thr Asn Arg Ala Tyr
       210                 215                 220
   Ala Ile Ala Ser Ser Val Val Ser Phe Tyr Val Pro Leu Cys Ile Met
   225                 230                 235                 240
   Ala Phe Val Tyr Leu Arg Val Phe Arg Glu Ala Gln Lys Gln Val Lys
                   245                 250                 255
   Lys Ile Asp Ser Cys Glu Arg Arg Phe Leu Gly Gly Pro Ala Arg Pro
               260                 265                 270
   Pro Ser Pro Ser Pro Ser Pro Val Pro Ala Pro Ala Pro Pro Pro Gly
           275                 280                 285
   Pro Pro Arg Pro Ala Ala Ala Ala Ala Thr Ala Pro Leu Ala Asn Gly
       290                 295                 300
   Arg Ala Gly Lys Arg Arg Pro Ser Arg Leu Val Ala Leu Arg Glu Gln
   305                 310                 315                 320
   Lys Ala Leu Lys Thr Leu Gly Ile Ile Met Gly Val Phe Thr Leu Cys
                   325                 330                 335
   Trp Leu Pro Phe Phe Leu Ala Asn Val Val Lys Ala Phe His Arg Glu
               340                 345                 350
   Leu Val Pro Asp Arg Leu Phe Val Phe Phe Asn Trp Leu Gly Tyr Ala
           355                 360                 365
   Asn Ser Ala Phe Asn Pro Ile Ile Tyr Cys Arg Ser Pro Asp Phe Arg
       370                 375                 380
   Lys Ala Phe Gln Arg Leu Leu Cys Cys Ala Arg Arg Ala Ala Cys Arg
   385                 390                 395                 400
   Arg Arg Ala Ala His Gly Asp Arg Pro Arg Ala Ser Gly Cys Leu Ala
                   405                 410                 415
   Arg Ala Gly Pro Pro Pro Ser Pro Gly Ala Ala Ser Asp Asp Asp Asp
               420                 425                 430
   Asp Asp Val Val Gly Ala Thr Pro Pro Ala Arg Leu Leu Glu Pro Trp
           435                 440                 445
   Ala Gly Cys Asn Gly Gly Ala Ala Ala Asp Ser Asp Ser Ser Leu Asp
       450                 455                 460
   Glu Pro Cys Arg Pro Gly Phe Ala Ser Glu Ser Lys Val
   465                 470                 475

## Beschreibung

Die Erfindung betrifft neue Sequenzvarianten des menschlichen β1-Adrenozeptorgens und die Verwendung von Polymorphismen im menschlichen β1-Adrenozeptorgen zur Diagnose von Cardiomyopathien.

Cardiomyopathien sind Erkrankungen des Herzmuskels, die nicht als Folge arteriosklerotischer Gefäßerkrankungen, Widerstandserhöhungen im kleinen oder großen Kreislauf sowie von Herzfehlern aufzufassen sind. Insbesondere die dilatative Cardiomyopathie (DCM), eine Cardiomyopathie unbekannter Ursache, ist eine Krankheit, die durch eine linke ventrikulare Disfunktion verbunden mit beeinträchtigter Funktion des β-adrenergenen Rezeptors gekennzeichnet ist. Es wird allgemein vorausgesetzt, daß ein adrenergener Overdnve eine entscheidende Rolle bei der Entwicklung einer DCM spielt (Francis GS et al, The neuronal humoral axis in congrestive heart failure, *Ann Inter Med* 1984; 101:370-377). Solch erhöhter adrenergener Overdrive führt zu einer Fehlregulierung der β1-Adrenozeptor gesteuerten Signalübertragung (Bristow MR et al, β1- and β2-adrenic-receptor-subtypes m non-failing and failing ventricular myocardium: coupling of both receptor subtypes to muscle contrating and selective β1-receptor down-regulation in heart failure. *Circ Res* 1986; 59:297-309). Der menschliche β1-Adrenozeptor spielt somit eine wichtige Rolle in der Regulation von Funktionen des Herzens. Die Gründe, die zu einer DCM führen, sind jedoch noch unbekannt.

Der Erfindung lag deshalb die Aufgabe zugrunde, Varianten, Polymorphismen, Mutationen und resultierende Haplotypen in der DNA-Sequenz des menschlichen β1-Adrenozeptorgens zu ermitteln und deren Korrelationen mit Krankheitsdispositionen festzustellen. Ausgehend von diesen Korrelationen soll ein Verfahren zur Diagnose dieser Krankheitsdispositionen sowie ein System zur Therapeutika-Entwicklung für diese Krankheiten entwickelt werden.

Die Aufgabe wird gemäß den Ansprüchen gelöst, die Unteransprüche sind Vorzugsvarianten.

Es wurde gefunden, daß in der 5'-regulierenden Region der Sequenz des menschlichen β1-Adrenozeptorgens in der kodierenden Region mindestens bis zu sieben Mutationen vorhanden sind sowie weitere stumme Mutationen existieren. Es wurde ferner gefunden, daß insbesondere drei dieser genetischen Varianten in der kodierenden Region mit der Disposition für Cardiomyopathien, insbesondere der DCM korrelieren. Vorzugsweise korrelieren zwei Mutationen im N-termmalen Bereich des Gens mit einer DCM.

Gegenstand der Erfindung sind danach Sequenzen des menschlichen β1-Adrenozeptorgens, die mindestens eine Mutation in der Position 175 (G→T, Ala59Ser) aufweisen. Ferner können die Sequenzen mindestens eine weitere Mutation in den Positionen 145 (A→G, Ser49Gly), 1165 (G→C, Gly389Arg), 1195 (C→T, Arg399Cys), 1205 (A→G, His402Arg), 1210 (A→G, Thr404Ala) und/oder 1252 (C→G, Pro418Ala) enthalten, vorzugsweise handelt es sich hierbei um Sequenzen mit den Mutationen 175 (G→T, Ala59Ser) und 145 (A→G, Ser49Gly), 175 (G→T, Ala59Ser) und 1165 (G→C, Gly389Arg) oder 175 (G→T, Ala59Ser), 145 (A→G, Ser49Gly) und 1165 (G→C, Gly389Arg).

SEQ ID No. 1 zeigt die Basenfolge mit Mutationen an allen genannten Positionen.

Darüber hinaus existieren stumme Mutationen. In der Tabelle 1 sind alle Mutationen (missense und stumme) dargestellt:

**Tabelle 1**

| | Nukleinsäure-austausch | Aminosäure-austausch |
|---|---|---|
| 1 | A145G | Ser49Gly |
| 2 | G 175T | Ala59Ser |
| 3 | G1165C | Gly389Arg |
| 4 | C1195T | Arg399Cys |
| 5 | C 1198A | Arg400Arg |
| 6 | G1200A | |
| 7 | A1205G | His402Arg |
| 8 | A1209G | Ala403Ala |
| 9 | A1210G | Thr404A1a |
| 10 | C 1249A | Ala417Ala |
| 11 | G1251A | |
| 12 | C1252G | Pro418Ala |
| 13 | C1254T | |
| 14 | C1260G | Gly420Gly |
| 15 | A1266G | Pro422Pro |
| 16 | G1269C | Pro423Pro |
| 17 | C1278T | Gly426Gly |

Im Sinne der Erfindung haben sich als besonders bedeutsame Sequenzen für eine Korrelation zu einer DCM erwiesen:
- Sequenz mit der Mutation 145 G, (SEQ ID No. 2)
- Sequenz mit der Mutation 175 T, (SEQ ID No. 3)
- Sequenz mit der Mutation 1165 C (SEQ ID No. 4)
- Sequenz mit den Mutationen 145 G und 175 T, (SEQ ID No. 5)
- Sequenz mit den Mutationen 145 G und 1165 C, (SEQ ID No. 6)
- Sequenz mit den Mutationen 175 T und 1165 C (SEQ ID No. 7)
- Sequenz mit den Mutationen 145 G, 175 T und 1165 C (SEQ ID No. 8).

Gegenstand der Erfindung ist deshalb ferner ein Verfahren zur Bestimmung von Krankheitsdispositionen, wobei die DNA des β1-Adrenozeptorgens eines Probanden mindestens an der Position 175 sowie gegebenenfalls an weiteren ausgetauschten Positionen genotypisiert und nachfolgend mit einer Referenz-DNA-Sequenz verglichen wird. Dabei können gegebenenfalls alle möglichen Kombinationen von Varianten von der Einzelmutation bis zu allen möglichen Kombinationen aller Varianten einschließlich jeder beliebigen Anzahl von Varianten einbezogen werden.

Bevorzugt sind Ausführungsformen, in denen die DNA neben der Position 175 noch mindestens an einer der Positionen 145, 1165, 1195, 1205, 1210 und/oder 1252 genotypisiert wird. Vorzugsweise wird die Genotypisierung durchgeführt an den Positionen 175 und 145, 175 und 1165 oder 175, 145 und 1165.

Die Genotypisierung erfolgt durch Sequenzierung oder durch andere Methoden, die für den Nachweis von Varianten (Punktmutationen) geeignet sind. Dazu gehören PCR-gestützte Genotypisierungsverfahren wie z. B. allelspezifische PCR, andere Genotypisierungsverfahren unter Verwendung von Oligonukleotiden sowie Verfahren unter Verwendung von Restriktionsenzymen.

Ausgehend davon ist das erfindungsgemäße Verfahren zur Bestimmung von Krankheitsdispositionen geeignet. In einer Ausführungsvariante z.B. zur Bestimmung einer Disposition für Cardiomyopathien, insbesondere von DCM.

Ferner kann das erfindungsgemäße Verfahren zur Bestimmung des Verlaufs und des Schweregrads von Cardiomyopathien, insbesondere von DCM herangezogen werden.

Ein weiterer wichtiger Gegenstand der Erfindung ist die Verwendung der beanspruchten Sequenzvananten zur Entwicklung von Therapeutika.

So sind sie bevorzugt zur Entwicklung von β1-Adrenozeptoragonisten und -antagonisten, zum Aufbau von Genen bzw. Vektoren, insbesondere zur Entwicklung von pharmazeutisch relevanten Substanzen sowie zur Entwicklung eines diagnostischen Kits verwendbar. Solche Kits können vorteilhaft zur Vorhersage der individuellen Ansprechbarkeit auf verschiedene β1-Rezeptorenblocker und zur Vorhersage der unterschiedlichen Disposition für Arzneimittelnebenwirkungen bzw. zur Vorhersage der Gewöhnung auf Medikamentengabe (Tachyphylaxie) eingesetzt werden.

Kulturen (Zellen), die die genannten unterschiedlichsten Kombinationen von individuellen β1-Varianten exprimieren, können somit als Testmodelle für die Entwicklung individuell spezifischer Therapeutika (β1-Liganden=Agonisten+Antagonisten) dienen. Das entspricht Testmodellen in vitro, aber auch in vivo Testmodelle (transgenic animals harboring these variant receptors).

Als individuelle Testmodelle erlauben sie in vitro (=ex vivo) eine Vorhersage zum individuellen Funktionszustand des β1-Adrenozeptorgens (β1-AR) bzw. der von ihm vermittelten Funktionen.

Der Umfang der beanspruchten Erfindung wird im folgenden ausführlich dargestellt.
Zur Erarbeitung der Erfindung wurde die gesamte bekannte DNA-Sequenz des menschlichen β1-Adrenozeptorgens einschließlich seiner regulierenden und kodierenden Regionen in Patienten und Kontrollen mittels der 'Multiplex PCR Sequenzierung' untersucht, und zunächst eine Reihe von genetischen Varianten identifiziert.

In der 5' regulierenden Region wurden zum bestehenden Zeitpunkt sieben neue Varianten entdeckt, deren wichtigste der Austausch von Ser → Gly (Position 49 im Protein) und Ala → Ser (Position 59) zum Nachweis einer Cardiomyopathie zu sein scheint.

Gegenstand der Erfindung sind deshalb auch neue Varianten des β₁-Adrenoreceptors.
Insbesondere handelt es sich um Proteine abgeleitet vom β₁-Adrenoreceptor mit Sequenzen, die neben der Mutation in der Position 175 (G→T) ganz oder teilweise ausgetauschte Aminosäuren in den Positionen 399 Arg→Cys, 402 His→Arg, 404 Thr→Ala und/oder 418 Pro→Ala aufweisen.

SEQ ID No. 9 zeigt das Protein mit einem Aminosäureaustausch an allen genannten Positionen.

Die Erfindung wird anschließend durch ein Ausführungsbeispiel näher erläutert.

37 Patienten (34 männlich, 3 weiblich), deren Alter von 23 bis 68 betrug und für die die Diagnose idiopathische dilatative Cardiomyopathie vorlag, wurden untersucht und mit Blutproben von 40 Probanden, bei denen keine Symptome von Herzkrankheiten erkennbar waren, verglichen.

Die DNA der DCM-Patienten und gesunden Probanden wurde aus Blutproben isoliert und auf Mutationen innerhalb der kodierenden Region und der 5' und 3' untranslatierten Region des β1-AR untersucht.

Für die Erhebung des gesamten polymorphen Spektrums des β1-Adrenozeptorgens wurde die PCR-Sequenziermethode verwendet. Hierzu wurde die gesamte bekannte Region in acht Fragmente unterteilt und mittels PCR amplifiziert. Die Sequenzierungsbedingungen (Primersequenzen, Annealing Temperatur, GC-Kit-Verwendung) sind in Tabelle 2 dargestellt. Es werden die GC-reichen Sequenzen unter Verwendung des Advantage-GC-Genomic-Polymerase-Mix (Clontech Lab. Inc.) entsprechend dem Protokoll des Herstellers amplifiziert. Die übrigen Assays werden unter Verwendung einer Mixtur enthaltend 50 mmol/l KCl, 1,5 mmol/l MgCl₂, 10 mmol/l Tris-HCl (pH = 8,3 bei 20 °C), 0,2 mmol/l von jedem Desoxynukleotid, 15 pmol/l des Primers und 1 U Taq DNA-Polymerase (Boehringer Mannheim) durchgeführt. Die Reaktionen fanden in einem Thermal-Cykler (Biometra) beginnend mit einem initiierten Denaturierungsschritt bei 95 °C für 3 Minuten statt.

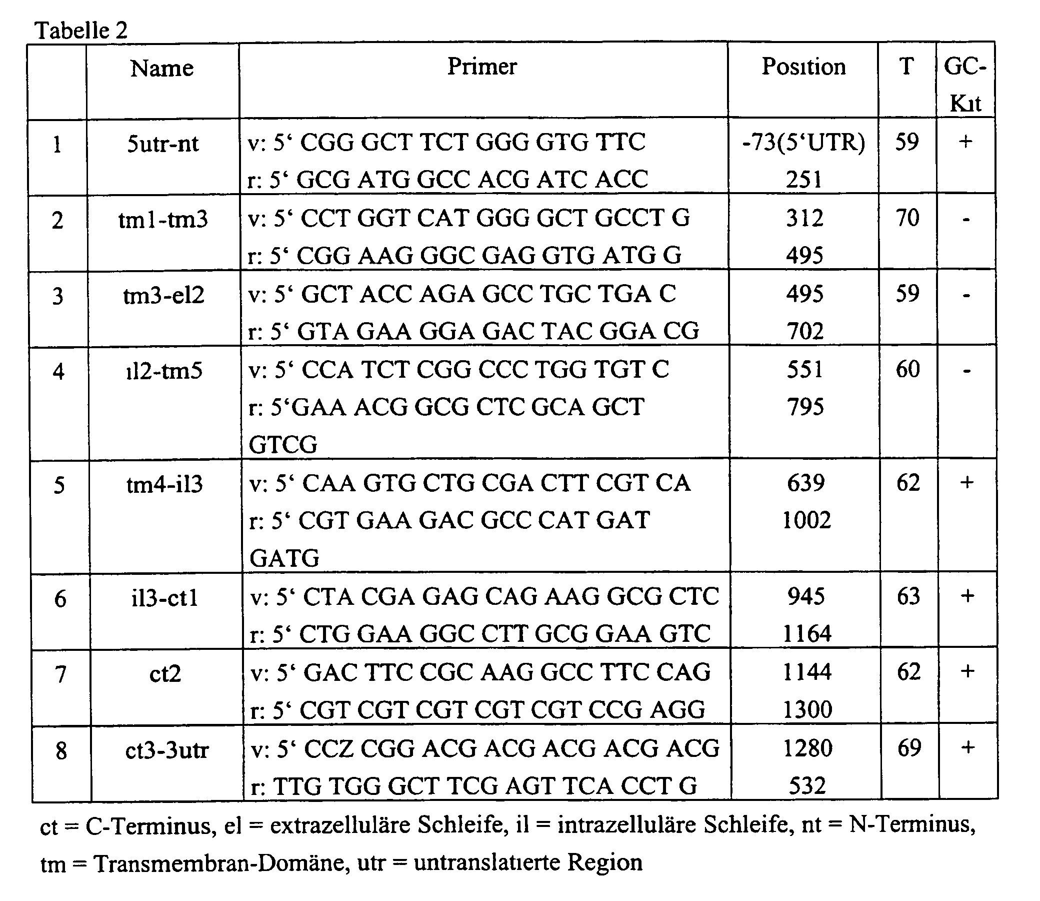

Die Mutationen wurden unter Anwendung der SSCP (single strang conformation polymorphism analysis) ausgewertet. Zur Identifizierung der Nukleinsäuresubstitutionen wurde ein automatisches Sequenzierungssystem (Applied Biosystem Incorporation) benutzt.

Es wurden sieben unterschiedliche Polymorphismen in der kodierenden Region des Gens gefunden, die der Tabelle 3 entnommen werden können.

**Tabelle 3**

| | Nukleinsäure-Substitution | Aminosäure-Substitution | Allel-Frequenz + Gesunde | Allel-Frequenz + DCM-Patienten |
|---|---|---|---|---|
| 1 | A145G | Ser49Gly | 0 | 0,095 |
| 2 | G175T | Ala59Ser | 0,38 | 0,108 |
| 3 | G1165C | Gly389Arg | 0.743 | 0,73 |
| 4 | C1195T | Arg399Cys | 0,014 | 0,027 |
| 5 | A1205G | His402Arg | 0,014 | 0,027 |
| 6 | A1210G | Thr404Ala | 0,014 | 0,027 |
| 7 | C1252G | Pro418Ala | 0,014 | 0,027 |

Zwei der dargestellten Substitutionen waren in der N-terminalen Region des β1-AR. Die Substitution der Nukleinsäure 145 (A → G) ergibt die Aminosäurealteration von Ser49 in Gly. Diese Mutation liegt überraschenderweise nur in DCM-Patienten vor (Allel-Frequenz 0,095, ein homozygoter Fall) und nicht bei gesunden Probanden.

Der zweite Aminosäureaustausch in der N-terminalen Region (Ala59 m Ser) wird durch eine Mutation der Nukleinsäure 175 (G → T) hervorgerufen, wobei die Substitution von Ala59Ser in DCM-Patienten wesentlich häufiger auftritt als in gesunden Probanden, ein Patient war homozygot.

Die weiteren Polymorphismen 3 bis 7 sind im cytoplasmatischen Ende in der Nähe der siebenten Transmembran Region des β1-AR lokalisiert. Der Polymorphismus in Position 1165 (G → C) bewirkt einen Aminosäurewechsel von Gly zu Arg. Diese Mutation kommt in der heterozygoten und homozygoten Form vor und ist so häufig vorhanden, daß in der Population Arginin die predominante Aminosäure ist.

Zusammenfassend ist festzustellen, daß für die zwei Mutationen innerhalb des N-Terminus des β1-AR die Allelfrequenz höher in DCM-Patienten war als bei den gesunden Testpersonen. Die Ser49Gly Aminosäuresubstitution war in 16,2 % der DCM-Patienten erkennbar. In den Kontrollen befand sich kein Glycin-Allel.
Die Ala59Ser Mutation war ebenfalls höher frequentiert in DCM-Patienten verglichen mit den Kontrollen.

Die Polymorphismen 3 bis 7 sind sämtlich am C-terminalen Ende von β1-AR lokalisiert. Die Gly389Arg Mutation (1165 G → C) hat eine Allel-Frequenz von 0,743 in gesunden Testpersonen und 0,73 in DCM-Patienten.
Die anderen vier Mutationen waren ausgesprochen selten vorhanden, und unterschieden sich kaum zwischen DCM-Patienten und gesunden Probanden. Der Polymorphismus in Position 1165 bewirkt einen Aminosäurewechsel von Gly in Arg. Diese Mutation erfolgte in der heterozygoten und in der homozygoten Form und war so oft vorhanden, daß in der untersuchten Population die prädominante Aminosäure Arginin ist.

## Patentansprüche

1. Sequenz des menschlichen β1-Adrenozeptorgens, **dadurch gekennzeichnet, daß** sie mindestens eine Mutation in der Position 175 (G→T) aufweist.

2. Sequenz nach Anspruch 1, **dadurch gekennzeichnet, daß** sie mindestens eine weitere Mutation in den Positionen 145 (A→G), 1165 (G→C), 1195 (C→T), 1205 (A→G), 1210 (A→G) und/oder 1252 (C→G) enthält.

3. Sequenz nach Anspruch 1 oder 2, **gekennzeichnet durch** die Mutationen in den Positionen 175 (G→T) und 145 (A→G).

4. Sequenz nach Anspruch 1 oder 2, **gekennzeichnet durch** die Mutationen in den Positionen 175 (G→T) und 1165 (G→C).

5. Sequenz nach Anspruch 1 oder 2, **gekennzeichnet durch** die Mutationen in den Positionen 175 (G→T), 145 (A→G) und 1165 (G→C).

6. Verfahren zur Bestimmung von Krankheitsdispositionen,
**dadurch gekennzeichnet, daß** die DNA des β1-Adrenozeptorgens eines Probanden mindestens an der Position 175 (G→T) sowie ggf. an weiteren ausgetauschten Positionen genotypisiert und nachfolgend mit einer Referenz-DNA-Sequenz verglichen wird, wobei ggf. alle möglichen Kombinationen von Varianten von der Einzelmutation bis zu allen möglichen Kombinationen aller Varianten einschließlich jeder beliebigen absoluten Anzahl von Varianten einbezogen werden.

7. Verfahren nach Anspruch 7, **dadurch gekennzeichnet, daß** die DNA mindestens an der Positionen 175 genotypisiert wird.

8. Verfahren nach Anspruch 7 oder 8, **dadurch gekennzeichnet, daß** die DNA zusätzlich mindestens an einer der Positionen 145, 1165, 1195, 1205, 1210 und/oder 1252 genotypisiert wird.

9. Verfahren nach Anspruch 7 bis 9, **dadurch gekennzeichnet, daß** die DNA mindestens an den Positionen 175 und 145 genotypisiert wird.

10. Verfahren nach Anspruch 7 bis 9, **dadurch gekennzeichnet, daß** die DNA mindestens an den Positionen 175 und 1165 genotypisiert wird.

11. Verfahren nach Anspruch 7 bis 9, **dadurch gekennzeichnet, daß** die DNA mindestens an den Positionen 175, 145 und 1165 genotypisiert wird.

12. Verfahren nach einem der Ansprüche 7 bis 12, **dadurch gekennzeichnet, daß** die Genotypisierung durch Sequenzierung oder durch andere Methoden, die für die Detektion von Vananten geeignet sind, erfolgt.

13. Verfahren nach einem der Ansprüche 7 bis 12 zur Bestimmung einer Disposition für Cardiomyopathien, insbesondere zur Bestimmung der dilatativen Cardiomyopathie.

14. Verfahren nach einem der Ansprüche 7 bis 12 zur Bestimmung des Verlaufs und des Schweregrads von Cardiomyopathien, insbesondere zur Bestimmung der dilatativen Cardiomyopathie.

15. Verwendung der Sequenzen gemäß einem der Ansprüche 1 bis 5 zur Entwicklung von Therapeutika, insbesondere zur Entwicklung einer neuen Klasse von β1-Adrenozeptoragonisten und -antagonisten.

16. Verwendung nach Anspruch 15 zum Aufbau von Genen bzw. Vektoren, insbesondere zur Entwicklung von pharmazeutisch relevanten Substanzen.

17. Verwendung nach Anspruch 15 oder 16 zur Entwicklung eines diagnostischen Kits.

18. Verwendung nach Anspruch 17 zur Entwicklung eines diagnostischen Kits zur Vorhersage der individuellen Ansprechbarkeit auf verschiedene β1-Adrenozeptor-antagonisten sowie - agonisten.

19. Verwendung nach Anspruch 17 oder 18 zur Entwicklung eines diagnostischen Kits zur Vorhersage der unterschiedlichen Disposition für Arzneimittelnebenwirkungen.

20. Verwendung nach Anspruch 19 oder 20 zur Entwicklung eines diagnostischen Kits zur Vorhersage der Gewöhnung auf Medikamentengabe (Tachyphylaxie).

21. Neue Varianten des β₁-Adrenorezeptors **gekennzeichnet durch** die Sequenzen, die neben der Mutation in der Position 175 (G→T) ganz oder teilweise ausgetauschte Aminosäuren in den Positionen 399 Arg→Cys, 402 His→Arg, 404 Thr→Ala und/oder 418 Pro→Ala aufweisen.

## Claims

1. Sequence of the human β1 adrenoceptor gene, wherein it manifests at least one mutation in position 175 (G→T).

2. Sequence according to Claim 1, wherein it contains at least one further mutation in positions 145 (A→G), 1165 (G→C), 1195 (C→T), 1205 (A→G), 1210 (A→G) and/or 1252 (C→G).

3. Sequence according to Claim 1 or 2, wherein it has the mutations in the positions 175 (G→T) and 145 (A→G).

4. Sequence according to Claim 1 or 2, wherein it has the mutations in the positions 175 (G→T) and 1165 (G→C).

5. Sequence according to Claim 1 or 2, wherein it has the mutations in the positions 175 (G→T), 145 (A→G) and 1165 (G→C).

6. Method for the determination of illness dispositions,
wherein the DNA of the β1 adrenoceptor gene of a test person is genotyped at least in position 175 (G→T) and, if need be, at further exchanged positions and is subsequently compared with a reference DNA sequence with, if need be, all possible combinations of variants of individual mutation right down to all possible combinations of all variants, including any arbitrary absolute quantity of variants, being included.

7. Method according to Claim 7, wherein the DNA is genotyped at least at position 175.

8. Method according to Claim 7 or 8, wherein in the DNA is additionally genotyped at least in one of the positions 145, 1165, 1195, 1205, 1210 and/or 1252.

9. Method according to Claims 7 to 9, wherein the DNA is genotyped at least in positions 175 and 145.

10. Method according to Claims 7 to 9, wherein the DNA is genotyped at least in positions 175 and 1165.

11. Method according to Claims 7 to 9, wherein the DNA is genotyped at least in positions 175, 145 and 1165.

12. Method according to one of the Claims 7 to 12, wherein the genotyping is done by sequencing or by other methods suitable for the detection of variants.

13. Method according to one of the Claims 7 to 12 for determination of a disposition for cardiomyopathies, in particular for determination of dilatative cardiomyopathy.

14. Method according to one of the Claims 7 to 12 for determination of the sequence and the degree of severity of cardiomyopathies, in particular for determination of dilatative cardiomyopathy.

15. Use of the sequences according to one of the Claims 1 to 5 for development of therapeutics, in particular for the development of a new class of β1 adrenoceptor agonists and antagonists.

16. Use according to Claim 15 for the build-up of genes or vectors, in particular for the development of pharmaceutically relevant substances.

17. Use according to Claim 15 or 16 for the development of a diagnostic kit.

18. Use according to Claim 17 for the development of a diagnostic kit for forecasting the individual reaction to various β1 adrenoceptor antagonists and agonists.

19. Use according to Claim 17 or 18 for the development of a diagnostic kit for forecasting the varying disposition for side-effects of drugs.

20. Use according to Claim 17 or 18 for the development of a diagnostic kit for forecasting adaptation to administration of drugs (tachyphylaxis).

21. New variants of the β₁ adrenoreceptor wherein there are the sequences manifesting not only the mutation in position 175 (G→T), but also totally or partly replaced amino acids in positions 399 Arg→Cys, 402 His→Arg, 404 Thr→Ala and/or 418 Pro→Ala.

## Revendications

1. Séquence du gène récepteur adrénergique β1 humain, **se caractérisant par le fait qu'**elle présente une mutation dans la position 175 (G→T).

2. Séquence selon la revendication 1, **se caractérisant par le fait qu'**elle contient au moins une autre mutation dans les positions 145 (A→G), 1165 (G→C), 1195 (C→T), 1205 (A→G), 1210 (A→G) et/ou 1252 (C→G).

3. Séquence selon la revendication 1 ou 2, **se caractérisant par** les mutations dans les positions 175 (G→T) et 145 (A→G).

4. Séquence selon la revendication 1 ou 2, **se caractérisant par** les mutations dans les positions 175 (G→T) et 1165 (G→C).

5. Séquence selon la revendication 1 ou 2, **se caractérisant par** les mutations dans les positions 175 (G→T), 145 (A→G) et 1165 (G→C).

6. Procédé permettant de déterminer des dispositions aux maladies,
**se caractérisant par le fait que** l'ADN du gène du récepteur adrénergique β1 d'un sujet test est génotypé au moins à la position 175 (G→T) ainsi qu'à d'autres positions changées le cas échéant et est ensuite comparé avec une séquence d'ADN de référence, ceci en y intégrant si nécessaire toutes les combinaisons possibles de variantes, de la mutation simple à toutes les combinaisons possibles de toutes les variantes, y compris d'un nombre absolu quelconque de variantes.

7. Procédé selon la revendication 7, **se caractérisant par le fait que** l'ADN est génotypé au moins sur la position 175.

8. Procédé selon la revendication 7 ou 8, **se caractérisant par le fait que** l'ADN est génotypé en plus au moins sur l'une des positions 145, 1165, 1195, 1205, 1210 et/ou 1252.

9. Procédé selon la revendication 7 à 9, **se caractérisant par le fait que** l'ADN est génotypé au moins sur les positions 175 et 145.

10. Procédé selon la revendication 7 à 9, **se caractérisant par le fait que** l'ADN est génotypé au moins sur les positions 175 et 1165.

11. Procédé selon la revendication 7 à 9, **se caractérisant par le fait que** l'ADN est génotypé au moins sur les positions 175, 145 et 1165.

12. Procédé selon l'une des revendications 7 à 12, **se caractérisant par le fait que** le génotypage se fait par séquençage ou par d'autres méthodes qui sont appropriées à la détection de variantes.

13. Procédé selon l'une des revendications 7 à 12 permettant de déterminer une disposition aux cardiomyopathies, permettant en particulier de déterminer la cardiomyopathie dilatatrice.

14. Procédé selon l'une des revendications 7 à 12 permettant de déterminer l'évolution et le degré de gravité de cardiomyopathies, permettant en particulier de déterminer la cardiomyopathie dilatatrice.

15. Emploi des séquences selon l'une des revendications 1 à 5 permettant de développer des médicaments, permettant en particulier de développer une nouvelle classe d'agonistes et d'antagonistes de récepteurs adrénergiques β1.

16. Emploi selon la revendication 15 permettant de constituer des gènes voire des vecteurs, permettant en particulier de développer des substances d'importance pharmaceutique.

17. Emploi selon la revendication 15 ou 16 permettant de développer un kit diagnostic.

18. Emploi selon la revendication 17 permettant de développer un kit diagnostic destiné à pronostiquer la réceptivité individuelle à différents antagonistes et agonistes de récepteurs adrénergiques β1.

19. Emploi selon la revendication 17 ou 18 permettant de développer un kit diagnostic destiné à pronostiquer la disposition différenciée aux effets secondaires résultant de la prise de médicaments.

20. Emploi selon la revendication 17 ou 18 permettant de développer un kit diagnostic destiné à pronostiquer l'accoutumance à la prise de médicaments (tachyphylaxie).

21. Nouvelles variantes du récepteur adrénergique β₁, **se caractérisant par** les séquences qui présentent en plus de la mutation dans la position 175 (G→T), des acides aminées intégralement ou partiellement changés dans les positions 399 Arg→Cys, 402 His→Arg, 404 Thr→Ala et/ou 418 Pro→Ala.
